# EUROPEAN PATENT SPECIFICATION

(11) **EP 1 800 712 B1**
(45) Date of publication and mention of the grant of the patent: **29.06.2011**
(21) Application number: 06077124.3
(22) Date of filing: 29.11.2006
(51) Int. Cl.: A61N 5/10

(54) **Apparatus for introducing catheters or needles into a body part**
Vorrichtung zur Einführung von Kathetern oder Nadeln in einen Körperteil
Appareil pour l'introduction de cathéters ou d'aiguilles dans une partie du corps

(30) Priority: 29.11.2005 NL 1030553
(43) Date of publication of application: 27.06.2007
(73) Proprietor: Isodose Control Intellectual Property B.V., 3905 TH Veenendaal (NL)
(72) Inventor: Van 't Hooft, Eric, 2930 Brasschaat (BE)
(74) Representative: van Loon, C.J.J.

(56) References cited:
- EP-A- 1 374 951
- US-A1- 2002 177 807

## Description

The invention relates to an apparatus for introducing one or more catheters or needles into a body part.

With some therapeutic treatments, in particular irradiating patients for the treatment of tumors, it is necessary to provide a plurality of catheters in the tissue. To this end, the conventional apparatus as, for instance, described in Rep1374951 provides a plate part, provided with a number of openings for guiding the catheters and/or needles. Other openings in the plate part may be used to suture the apparatus to the skin of the patient.

With the treatment of prostate tumors in men, for instance, the conventional use of this apparatus, also referred to as template, is to fix the plate part against the skin and to insert the needles into the prostate with the aid and with guidance of the plate part. After placing the needles, the needles are clamped in the plate part or a plate part is used which makes it difficult for the needles to move.

Due to the fact that the plate part is fixed on the skin, with swelling of the tissue - often precisely caused by introducing the needles - the skin will press against the plate part so that the plate part with needles can be pressed away from the prostate up to as much as 20 mm.

This can give rise to an undesired situation which can result in a wrong irradiation. In order to correct this, the needles need to be pressed back again regularly or before each repeated irradiation, often with diagnostic guidance. This operation is not only very time-consuming, but also particularly unpleasant and even painful for the patient. Also, as a result of this, often extra anesthetics need to be given to the patient.
It is an object of the invention to be able to offer an alternative for above-mentioned treatment method and, to this end, it provides an apparatus according to claim 1. In particular, the invention provides an apparatus for introducing one or more catheters or needles into a body part, comprising a plate part, provided with a first coupling mechanism for coupling and guiding the catheters and/or needles; and a second coupling mechanism, different from the first coupling mechanism. The first coupling mechanism comprises a plurality of first openings provided in the plate part, for guiding the catheters and/or needles; and the second coupling mechanism comprises at least one opening, dimensioned to correspond with the at least one anchoring needle in the plate part. A clamp is provided for clamping the anchoring needle in the opening for anchoring the anchoring needle in the body part for fixation of the plate part with respect to the body part

As a result, the template can be placed at a large distance from the skin and the anchoring needles fix the template with respect to the skin. Such anchoring needles known per se typically have an anchoring mechanism for anchoring in the tissue. Typically, such needles may be provided with a double wall of which the outer wall has multiple parallel indentations all round so that, upon pulling the inner catheter, the outer wall bends outwards at the location of the indentations so that this catheter locks in the tissue.

Due to fixation of the template by means of these anchoring needles, further, standard catheters can be fixed to the plate part so that the plate part fixes all locked catheters in the same place in the prostate.

The invention will be explained in more detail with reference to the description of the drawings, in which:
Fig. 1 shows a conventional catheter;
Fig. 2 shows an anchoring needle;
Fig. 3 shows an embodiment of the apparatus according to the invention;
Fig. 4 shows a fragment of a locking mechanism for locking the anchoring needle; and
Fig. 5 shows a side elevational view of the apparatus in use.

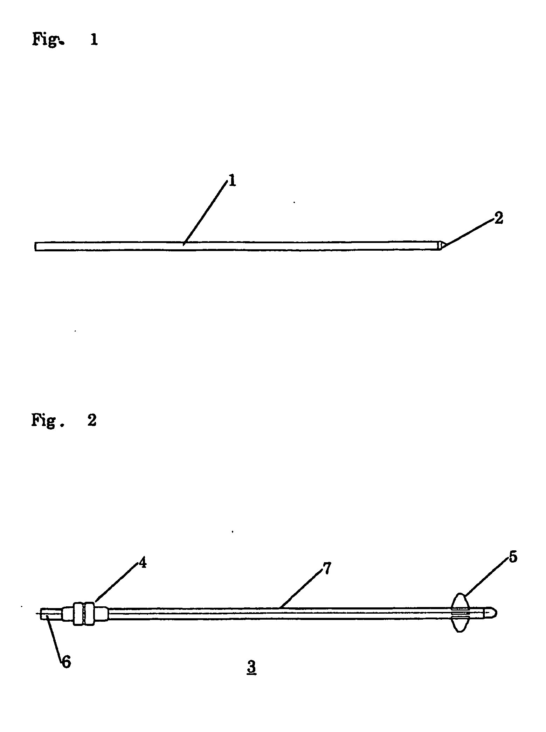

Fig. 1 shows a conventional catheter 1. The catheter 1 contains a conventional catheter needle 2 which is fed through the catheter 1, which needle 2 can be inserted into the tissue with catheter 1. After providing the needle 2 in the tissue, it is removed from the catheter 1 by, for instance, withdrawing it at the back side, and the catheter 1 can be connected to a brachytherapy apparatus (not shown), which feeds a radioactive source through the catheter 1 to the position to be irradiated in the tissue. The conventional brachytherapy provides the placement of a number of catheters in a tissue to be irradiated in predetermined positions, such that the tissue receives a predetermined irradiation dose from radioactive sources which are provided in the catheters. To this end, normally use is made of a plate part (not shown), which is provided on the skin and fixes the catheters. As discussed hereinabove, this has the drawback that, with swelling of the skin and underlying tissues, the position of the catheters in the tissue can be disturbed, which has an adverse effect on the effectiveness of the irradiation.

Fig. 2 shows a conventional anchoring needle 3 which can be used for fixation in a tissue. The anchoring needle 3 typically comprises a fixing part 4 and a laterally movable anchoring element 5 to be anchored in the body part, which element can be fixed by rotation or displacement of the fixing part. This may, for instance, be achieved by means of a central part 6, which can be moved in a tube part 7 and which presses the anchoring element 5, formed by a strip-shaped structure provided in the tube part, laterally outwards upon displacement. Fig. 2 shows the position pressed outwards of the anchoring element 5.

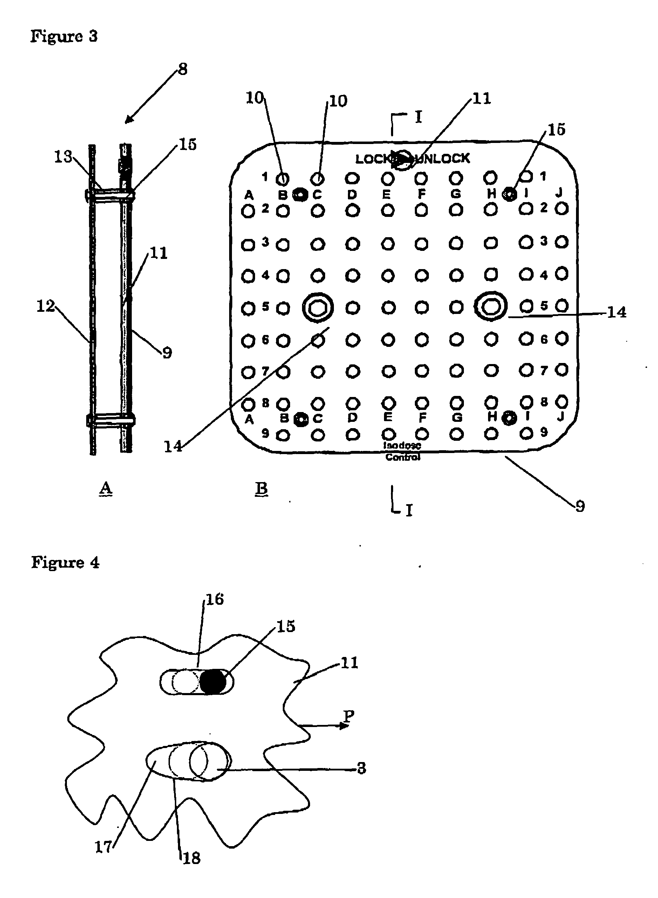

Fig. 3 shows the template apparatus 8 according to the invention. In particular, part A shows the apparatus in cross section, viewed along the line I-I in part B, which shows a top plan view of the apparatus 8. According to Fig. 3, the apparatus comprises a plate part 9 with conventional guide openings 10 as a first coupling mechanism for guiding catheters and/or needles (not shown). A clamping plate 11 is provided for locking the needles in the guide openings 10. A further plate part 12 is provided, kept at a distance from plate part 9 by spacers 13. The plate parts are manufactured from a relatively light material, such as a plastic, and thus provide a linear guiding of the catheters and/or needles.

The template 8 further comprises two guide openings 14 for an anchoring needle 3 shown in Fig. 2. The guide openings 14 are centrally placed, and form a coupling mechanism different from the first mechanism, with clamping plate 11 for coupling the anchoring needle 3. Alternatively or in addition, a distinct fixing mechanism may be present to fix the anchoring needle 3. Thus, by anchoring of the anchoring needles 3 in a body part, the plate part 9 can be fixed with respect to the body part.

In the example of the drawing, to this end, two openings 14 are provided in central positions symmetrically with respect to the center. In other embodiments, a triangular structure may, for instance, also be used to anchor the template 8 with three anchoring needles 3. In one variant, the anchoring needle 3 is provided with a hollow structure through which a radioactive source can be guided. As shown, the coupling mechanism is formed by the clamping plate 11 in cooperation with the guide openings 14. This coupling mechanism further provides fixing of catheters and/or needles as shown in Fig. 1, by clamping them in openings 10. The clamping plate 11 is fixed to the plate part 9 with guides 15.

Fig. 4 schematically shows a fragment of the clamping plate 11. Here, a guide 15 can slidingly move in a slot 16. As a result, the clamping plate 11 moves relative to the plate part 9. A catheter 1 or anchoring needle 3 put through an opening 10 or 14 of the plate part 9 runs through the clamping plate 11, in particular through the opening 17. The opening comprises sides 18 which form a running surface which clampingly abuts a catheter 1 put through an opening of the first plate part 9. In the Figure, in continuous lines, a released position is drawn of the needle 3; and, in broken lines, the locked position realized by displacement in the direction of arrow P.

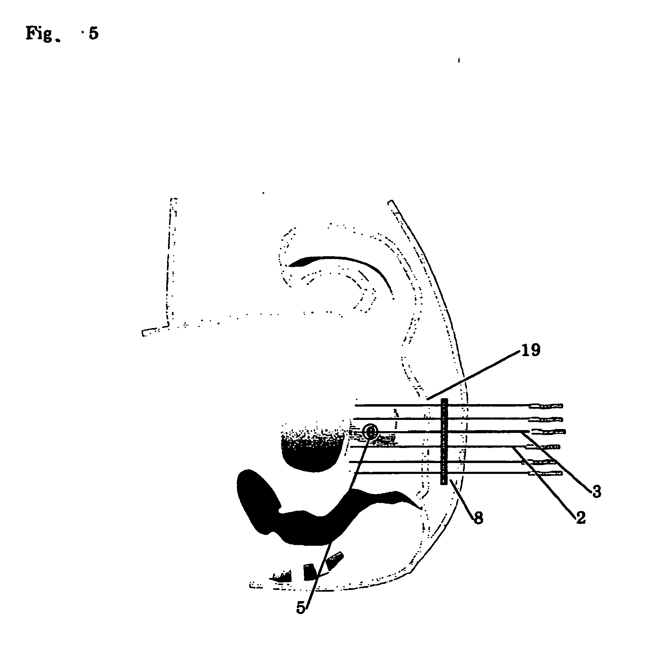

Fig. 5 shows a schematic representation of the apparatus in use. In particular, it can be seen how the template 8 is kept at a distance from the skin 19. The template is fixed with respect to the prostate by means of an anchoring needle 3, in particular the anchoring element 5 thereof. The anchoring needle 3 is coupled with the template. The template further provides guiding of catheters 2 and/or needles by means of the template 8 anchored on the prostate.

The invention is not limited to the embodiments shown in the drawing but may also comprise alternatives or variants thereof which fall within the scope of the following claims. Such variants may, for instance, comprise alternative coupling mechanisms, where the anchoring needle is coupled separately from the catheters. Also, a coupling construction may be provided, for instance, by means of a clip fastening or the like to fix the template 8 on an anchoring needle. The anchoring needle 3 itself may be designed in various manners, as long as an anchoring in the tissue is provided. Such variants are understood to fall within the claims defined in the following.

## Claims

1. An apparatus for introducing one or more catheters or needles into a body part, comprising:
- a plate part (9), provided with a first coupling mechanism (10) for coupling and guiding the catheters and/or needles (2); and
- a second coupling mechanism (14), different from the first coupling mechanism (10), wherein the first coupling mechanism comprises a plurality of first openings (10) provided in the plate part (9), for guiding the catheters and/or needles (2); and the second coupling mechanism comprises at least one second opening (14) in the plate part (9), **characterized in that** the second opening (14) is dimensioned to correspond with at least one anchoring needle (3), and **characterized by** a clamp (11) for clamping the anchoring needle (3) in the second opening, the anchoring needle (3) anchorable in the body part thereby enabling the fixation of the plate part (9) with respect to the body part.

2. An apparatus according to any one of the preceding claims, wherein the coupling mechanism further provides a fixing (11) of the catheters and/or needles to the plate.

3. An apparatus according to any one of the preceding claims, wherein the coupling mechanism comprises a clamping plate (11) which is, parallel to the plate part (9, 12), displaceable from a locking position to a releasing position, which clamping plate (11) makes a running surface clampingly abut a catheter or needle put through said first openings (10) of the plate part due to displacement.

4. An apparatus according to any one of the preceding claims, further comprising a further plate part (12, 13) and spacers, for keeping the further plate part (12) at a distance with respect to the plate part (9), in order to provide a linear guiding of the catheters and/or needles.

5. An assembly of an apparatus according to any one of the preceding claims and at least one anchoring needle (3) wherein the anchoring needle (3), corresponds with the second coupling mechanism (10).

6. An apparatus according to claim 5, wherein the anchoring needle (3), comprises a fixing part and a laterally movable anchoring element (5) to be anchored in the body part, which anchoring element can be fixed in the body part by the fixing part (4).

## Patentansprüche

1. Vorrichtung zur Einführung eines oder mehrerer Katheder oder einer oder mehrerer Nadeln in ein Körperteil, wobei die Vorrichtung aufweist:
- ein Plattenteil (9), das mit einem ersten Kopplungsmechanismus (10) zum Koppeln und Führen der Katheder und/oder Nadeln (2) ausgestattet ist, und
- einen zweiten Kopplungsmechanismus (14), der von dem ersten Kopplungsmechanismus (10) verschieden ist, wobei der erste Kopplungsmechanismus eine Vielzahl von in dem Plattenteil (9) vorgesehenen ersten Öffnungen (10) zum Führen der Katheder und/oder Nadeln (2) aufweist und der zweite Kopplungsmechanismus mindestens eine zweite Öffnung (14) in dem Plattenteil (9) aufweist, **dadurch gekennzeichnet, dass** die zweite Öffnung (14) so dimensioniert ist, dass sie mindestens einer Verankerungsnadel (3) entspricht, und eine Klemmeinrichtung (11) zum Klemmen der Verankerungsnadel (3) in der zweiten Öffnung vorhanden ist, wobei die Verankerungsnadel (3) in dem Körperteil verankerbar ist und **dadurch** die Befestigung des Plattenteils (9) in Bezug auf den Körperteil ermöglicht.

2. Vorrichtung nach einem der vorhergehenden Ansprüche, bei der der Kopplungsmechanismus ferner eine Befestigung (11) der Katheder und/oder Nadeln an der Platte bereitstellt.

3. Vorrichtung nach einem der vorhergehenden Ansprüche, bei der der Kopplungsmechanismus eine Klemmplatte (11) aufweist, die parallel zu dem Plattenteil (9, 12) aus einer Verriegelungsposition in eine Freigabeposition verlagerbar ist, wobei die Klemmplatte (11) dafür sorgt, dass eine Lauffläche aufgrund von Verlagerung klemmend an einem Katheder oder einer Nadel anliegt, die durch die ersten Öffnungen (10) des Plattenteils geführt sind.

4. Vorrichtung nach einem der vorhergehenden Ansprüche, die ferner ein weiteres Plattenteil (12, 13) und Abstandhalter zum Halten des weiteren Plattenteils (12) in einem Abstand in Bezug auf das Plattenteil (9) aufweist, um eine lineare Führung der Katheder und/oder Nadeln bereitzustellen.

5. Anordnung einer Vorrichtung nach einem der vorhergehenden Ansprüche und mindestens einer Verankerungsnadel (3), wobei die Verankerungsnadel (3) dem zweiten Kopplungsmechanismus (10) entspricht.

6. Vorrichtung nach Anspruch 5, bei der die Verankerungsnadel (3) ein Befestigungsteil und ein seitlich bewegbares Verankerungselement (5) aufweist, das in dem Körperteil zu verankern ist, wobei das Verankerungselement durch das Befestigungsteil (4) in dem Körperteil befestigt werden kann.

## Revendications

1. Appareil d'introduction d'un ou plusieurs cathéters ou aiguilles dans une partie du corps, comprenant :
- une plaque (9) avec un premier mécanisme de couplage (10) permettant le couplage et le guidage des cathéters et/ou des aiguilles (2) ; et
- un deuxième mécanisme de couplage (14), différent du premier mécanisme de couplage (10),
le premier mécanisme de couplage comprenant une pluralité de premières ouvertures (10) ménagées dans la plaque (9) destinées à guider les cathéters et/ou les aiguilles (2) ; et le deuxième mécanisme de couplage comprenant au moins une deuxième ouverture (14) ménagée dans la plaque (9), **caractérisé en ce que** la deuxième ouverture (14) est dimensionnée pour être associée à au moins une aiguille d'ancrage (3) et un dispositif de blocage (11) destiné à bloquer l'aiguille d'ancrage (3) dans la deuxième ouverture, l'aiguille d'ancrage (3) pouvant être ancrée dans la partie du corps, permettant ainsi d'immobiliser la plaque (9) par rapport à la partie du corps.

2. Appareil selon l'une quelconque des revendications précédentes, dans lequel le mécanisme de couplage prévoit en outre un dispositif de blocage (11) des cathéters et/ou des aiguilles par rapport à la plaque.

3. Appareil selon l'une quelconque des revendications précédentes, dans lequel le mécanisme de couplage comprend une plaque de blocage (11) pouvant être déplacée, parallèlement à la plaque (9, 12), d'une position de verrouillage à une position de déverrouillage, ladite plaque de blocage (11) provoquant, à la suite du déplacement, la coopération en appui et en blocage d'une portée contre un cathéter ou une aiguille traversant lesdites premières ouvertures (10) de la plaque.

4. Appareil selon l'une quelconque des revendications précédentes, comprenant également une plaque supplémentaire (12, 13) et des éléments d'espacement destinés à maintenir la plaque supplémentaire (12) à distance de la plaque (9), pour permettre un guidage linéaire des cathéters et/ou des aiguilles.

5. Ensemble formé d'un appareil selon l'une quelconque des revendications précédentes et d'au moins une aiguille d'ancrage (3), dans lequel l'aiguille d'ancrage (3) est associée au deuxième mécanisme de couplage (14).

6. Appareil selon la revendication 5, dans lequel l'aiguille d'ancrage (3) comprend un élément d'immobilisation et un élément d'ancrage mobile latéralement (5) destiné à être ancré dans la partie du corps, lequel élément d'ancrage peut être immobilisé dans la partie du corps par l'élément d'immobilisation (4).
